# EUROPÄISCHE PATENTSCHRIFT

(11) **EP 1 177 767 B1**
(45) Veröffentlichungstag und Bekanntmachung des Hinweises auf die Patenterteilung: **30.11.2005**
(21) Anmeldenummer: 01000347.3
(22) Anmeldetag: 03.08.2001
(51) Int. Cl.: A61B 6/03

(54) **Computertomograph mit kegelförmigem Strahlenbündel und helixförmiger Relativbewegung**
Computer tomograph with coneshaped beam and helicoidal relative movement
Tomographie par ordinateur à faisceau conique et mouvement relatif hélicoidal

(30) Priorität: 05.08.2000 DE 10038328
(43) Veröffentlichungstag der Anmeldung: 06.02.2002
(73) Patentinhaber: Philips Intellectual Property & Standards GmbH, 20099 Hamburg (DE); Koninklijke Philips Electronics N.V., 5621 BA Eindhoven (NL)
(72) Erfinder: Köhler, Thomas, Philips Corp. Intell. Prop. GmbH, 52064, Aachen (DE); Proksa, Roland, Philips Corp. Intell. Prop. GmbH, 52064, Aachen (DE)
(74) Vertreter: Volmer, Georg

(56) Entgegenhaltungen:
- DE-A- 19 832 276
- DE-A- 19 835 296
- DE-A- 19 905 975
- US-A- 5 105 087
- US-A- 5 570 403

## Beschreibung

Die Erfindung betrifft einen Computertomographen mit einer ein kegelförmiges Strahlenbündel emittierenden Strahlenquelle, die sich relativ zu einem Untersuchungsbereich bzw. einem darin befindlichen Objekt auf einer helixförmigen Bahn bewegt.

Bei einem aus der US 6,275,561 B1 bekannten Computertomographen dieser Art hat eine mit der Strahlenquelle verbundene Detektoreinheit, die von dem kegelförmigen Strahlenbündel nach dem Durchgang durch den Untersuchungsbereich getroffen wird, eine solche Form, dass ihre in axialer Richtung gegeneinander versetzten äußeren Ränder zwei benachbarte Windungen der Helix bedecken. Es lässt sich zeigen, dass dabei die Voxel im Untersuchungsbereich von der Strahlenquelle auf die Detektoreineit aus einem Winkelbereich von genau 180° projiziert werden. Aus den dabei gewonnenen CT-Daten (CT=Computertomographie) lässt sich ein CT-Bild mit guter Bildqualität rekonstruieren.

Eine noch bessere Bildqualität lässt sich mit einem aus der DE 198 35 296 A1 bekannten Computertomographen erzielen, bei dem die Abmessungen der Detektoreinheit in axialer Richtung so gewählt sind, dass die Projektion der äußeren Ränder der Detektoreinheit auf die Helix eine Strecke von (2n + 1) p einschließt, wobei p dem axialen Versatz zweier benachbarter Windungen der Helix entspricht. Jedes Voxel im Untersuchungsbereich wird dabei aus einem Winkelbereich von (2n +1)·180° auf die Detektoreinheit projiziert. Dadurch ist das Signal/Rauschverhältnis in den Voxeln gleichmäßiger über den Untersuchungsbereich verteilt als bei dem eingangs erwähnten Computertomographen. Außerdem kann das Signal/Rauschverhältnis (bei gleicher Relativgeschwindigkeit zwischen Strahlenquelle und Untersuchungsbereich) um einen Faktor 2n + 1 besser sein als bei dem bekannten Computertomographen; Voraussetzung dafür ist allerdings, dass auch die Abmessungen der Detektoreinheit in axialer Richtung um einen Faktor 2n + 1 größer sind.

Es ist jedoch nicht möglich, die Abmessungen bestimmter Detektoreinheiten in axialer Richtung beliebig zu vergrößern. Wenn die Detektoreinheit beispielsweise Halbleiterchips mit matrixförmig angeordneten Detektorelementen enthält, dann müssen die Halbleiterchips in einer zur Rotationsachse senkrechten Ebene bogenförmig aneinandergereiht werden. Da auf einer der vier Seiten eines Chips eine Elektronik erforderlich ist, die die Signale der Detektorelemente weiterverarbeitet, können in Richtung senkrecht zu der erwähnten Ebene, d. h. in axialer Richtung, nur zwei Chips unmittelbar nebeneinander angeordnet werden, wobei die mit den einzelnen Chips verbundene Elektronik sich jeweils auf den voneinander abgewandten Seiten der Chips befindet. Da die Größe der Chips aber aus fertigungstechnischen Gründen begrenzt ist, sind auch die Abmessungen der Detektoreinheit in axialer Richtung begrenzt.

Aufgabe der vorliegenden Erfindung ist es, einen Computertomographen zu schaffen, dessen Detektoreinheit trotz dieser Beschränkungen CT-Daten liefert, aus denen sich CT-Bilder mit verbessertem Signal/Rauschverhältnis und einer günstigeren räumlichen Verteilung des Signal/Rauschverhältnisses rekonstruieren lassen.

Diese Aufgabe wird erfindungsgemäß gelöst durch einen Computertomograph mit
- einer Abtasteinheit, die eine Strahlenquelle (S) und eine damit verbundene Detektoreinheit (16) zur Erfassung eines von der Strahlenquelle (S) emittierten,
- kegelförmigen Strahlenbündels nach dem Durchgang durch einen Untersuchungsbereich (13) bzw. durch ein darin befindliches Objekt umfasst,
- einer Antriebsanordnung (2, 5) zur Erzeugung einer eine Rotation um eine Rotationsachse (14) und einen Vorschub parallel zur Rotationsachse umfassenden Relativbewegung in Form einer Helix zwischen der Abtasteinheit (S, 16) und dem Untersuchungsbereich (13) bzw. dem Objekt,
- wobei die Projektion der in axialer Richtung gegeneinander versetzten äußeren Ränder der Detektoreinheit auf die Helix eine Strecke von (2n+1)p einschließt, wobei n eine ganze Zahl ≥ 1 ist und p dem axialen Versatz zweier benachbarter Windungen der Helix entspricht,
- wobei die Detektoreinheit mehrere räumlich voneinander getrennte und in axialer Richtung gegeneinander versetzte eine Vielzahl von Detektorelementen umfassende Detektorsegmente (161, 162) im Abstand voneinander umfasst, wobei zwischen den Detektorsegmenten (161, 162) ein Zwischenraum (18) verbleibt, und jedes Detektorsegment (161, 162) so angeordnet und gestaltet ist, dass seine Projektion auf die Helix mindestens zwei benachbarte Windungen der Helix bedeckt.

Die Erfindung nutzt Symmetrien bei der Datengewinnung mit dem bekannten Computertomographen aus. Wenn sich bei einem solchen Computertomographen die Projektion der in axialer Richtung gegeneinander versetzten äußeren Ränder der Detektoreinheit z.B. über das fünffache des Abstandes zweier benachbarter Windungen der Helix erstrecken, dann lässt sich eine solche Detektoreinheit gedanklich in fünf Detektorsegmente unterteilen, deren Projektion jeweils zwei benachbarte Windungen der Helix miteinander verbindet.

Auf das mittlere Detektorsegment werden die Voxel im Untersuchungsbereich von der Strahlenquelle aus einem Winkelbereich von 180° projiziert. Diese Daten sind ausreichend, um eine vollständige Rekonstruktion zu gewährleisten. Andererseits ist auch mit den CT-Daten der drei inneren Detektorsegmente, auf die die Voxel des Untersuchungsbereichs aus einem Winkelbereich von 3 mal 180° projiziert werden, eine Rekonstruktion (mit verbesserter Bildqualität) möglich. Schließlich ist auch eine Rekonstruktion aus den CT-Daten aller fünf Detektorsegmente (auf die die Voxel des Untersuchungsbereichs aus einem Winkelbereich von 5 mal 180° projiziert werden) möglich.

Da der Rekonstruktionsprozess, mit dem die CT-Daten zu einem (dreidimensionalen) CT-Bild verarbeitet werden, linear ist, bedeutet dies, dass man aus den CT-Daten der äußeren Detektorsegmente, des mittleren Detektorsegments und der dazwischen liegenden Detektorsegmente jeweils ein vollständiges CT-Bild rekonstruieren kann. Bei der Erfindung wird dies ausgenutzt, um einzelne Detektorsegmente bzw. Paare von Detektorsegmenten (z. B. die beiden jeweils zwischen dem mittleren und einem äußeren Detektorsegment liegenden Detektorsegmente) wegzulassen. Aus den CT-Daten der übrigen Detektorsegmente lässt sich dann immer noch ein CT-Bild rekonstruieren, das ein günstigeres Signal/Rauschverhältnis und eine homogenere räumliche Verteilung dieses Verhältnisses aufweist als ein CT-Bild, das lediglich aus den CT-Daten des mittleren Detektorsegments rekonstruiert wird.

Durch das Weglassen dieser Detektorsegmente ergeben sich zwischen einzelnen Detektorsegmenten freie Bereiche, in denen sich beispielsweise die Elektronik für die einzelnen Detektorsegmente oder Befestigungspunkte für die Detektorsegmente oder davor angeordnete Streustrahlenraster befinden können.

Die einfachste mögliche Detektoreinheit ist in Anspruch 2 beschrieben. Allerdings ergeben sich bei einer solchen Detektoreinheit Probleme, wenn statt einer helixförmigen Relativbewegung zwischen Untersuchungsbereich und Strahlenquelle eine kreisförmige Relativbewegung ausgeführt wird. Der in der Ebene zwischen den beiden Detektorsegmenten befindliche Teil des Untersuchungsbereichs lässt sich damit nicht frei von Artefakten rekonstruieren.

Insofern ist die in Anspruch 3 angegebene Ausgestaltung der Erfindung günstiger, die allerdings mindestens drei Detektorsegmente erfordert. Mit dem Detektorsegment, das sich in der Ebene der Strahlenquelle befindet, kann auch bei einer kreisförmigen Relativbewegung eine zentrale Schicht frei von den genannten Artefakten rekonstruiert werden.

Eine bevorzugte Ausgestaltung ist in Anspruch 4 angegeben. Damit wird bei einem für medizinische Zwecke benutzten Computertomographen die Strahlenbelastung reduziert.

Mit der Ausgestaltung nach Anspruch 5 können CT-Bilder für mehrere Energiebereiche rekonstruiert werden, wobei die dazu erforderlichen CT-Daten gleichzeitig akquiriert werden.

Die Erfindung wird nachstehend anhand der Zeichnungen näher erläutert. Es zeigen:
Fig. 1 einen erfindungsgemäßen Computertomographen in schematischer Darstellung,
Fig, 2 eine vereinfachte Draufsicht auf einen solchen Computertomographen,
Fig. 3 die geometrischen Verhältnisse bei einem solchen Computertomographen,
Fig 4 die Abwicklung einer erfindungsgemäßen Detektoreinheit und die Projektion der helixförmigen Windungen auf diese Einheit,
Fig. 5 eine dafür geeignete Blendenanoidnung,
Fig. 6 die geometrischen Verhältnisse bei einer zweiten Ausführungsform,
Fig 7 einen Querschnitt durch diese Ausführungsform in einer die Rotationsachse enthaltenden Ebene und
Fig. 8a und 8b die geometrischen Verhältnisse bei 2 weiteren Ausführungsformen der Erfindung

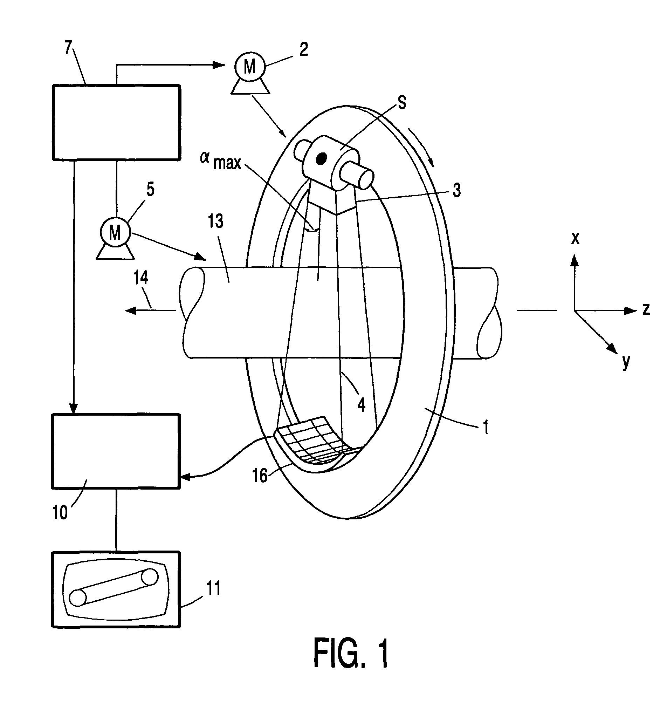

Der in Fig. 1 dargestellte Computertomograph umfasst eine Gantry 1, die um eine parallel zur z-Richtung des in Fig. 1 dargestellten Koordinatensystem verlaufende Rotationsachse 14 rotieren kann. Dazu wird die Gantry von einem Motor 2 mit einer vorzugsweise konstanten, aber einstellbaren Winkelgeschwindigkeit angetrieben. An der Gantry ist eine Strahlenquelle S befestigt, beispielsweise ein Röntgenstrahler. Dieser ist mit einer Kollimatoranordnung 3 versehen, die aus der von der Strahlenquelle S erzeugten Strahlung ein kegelförmiges Strahlenbündel 4 ausblendet, d. h. ein Strahlenbündel, das sowohl in z-Richtung als auch in einer dazu senkrechten Richtung (d. h. in einer zur Rotationsachse 14 senkrechten Ebene) eine von Null verschiedene endliche Ausdehnung hat. Wie weiter unten noch erläutert, kann das Strahlenbündel 4 sich aus mehreren in Richtung der Rotationsachse gegeneinander versetzten Strahlenkegeln zusammensetzen.

Das Strahlenbündel 4 durchdringt einen Untersuchungsbereich 13, in dem sich ein Objekt, z. B. ein Patient auf einem Patientenlagerungstisch (beides nicht näher dargestellt), befinden kann. Der Untersuchungsbereich 13 hat die Form eines Zylinders. Nach dem Durchsetzen des Untersuchungsbereichs 13 trifft das Röntgenstrahlenbündel 14 auf eine an der Gantry 1 befestigte zweidimensionale Detektoreinheit 16.

Die Detektoreinheit 16 besteht aus mehreren im Abstand voneinander angeordneten Segmenten - was in Fig 1 nicht näher dargestellt ist. In einer zur Rotationsachse 14 senkrechten Ebene beschreibt die Detektoreinheit einen Kreisbogen um die Strahlenquelle S. Sie kann aber auch anders geformt sein, z. B. einen Kreisbogen um die Rotationsachse 14 beschreiben oder geradlinig sein. Jedes Detektorsegment umfasst eine Vielzahl von Detektorelementen, die in jeder Position der Strahlenquelle einen Messwert für einen Strahl aus dem Strahlenbündel 4 liefern. Diese Messwerte werden im folgenden auch als CT-Daten bezeichnet. Die von der Detektoreinheit 16 akquirierten Messwerte bzw. CT-Daten werden einem Bildverarbeitungsrechner 10 zugeführt, der daraus die Absorptionswerteilung in einem Teil des Untersuchungsbereichs rekonstruiert und z B. auf einem Monitor 11 wiedergibt.

Der mit αₘₐₓ bezeichnete Öffnungswinkel des Strahlenbündels 4 (als Öffnungswinkel ist der Winkel definiert, den ein Strahl der in einer zur Rotationsachse 14 senkrechten Ebene am Rande des Strahlenbündels 4 liegt, mit einer durch die Strahlenquelle S und die Rotationsachse 14 definierten Ebene einschließt) bestimmt dabei den Durchmesser des zylinderförmigen Untersuchungsbereichs 13, innerhalb dessen sich das zu untersuchende Objekt bei der Akquisition der CT-Daten befindet.

Der Untersuchungsbereich 13 - bzw. das Objekt oder der Patientenlagarungstisch - kann mittels eines Motors 5 parallel zur Rotationsachse 14 bzw. parallel zur z-Achse geschoben werden. Dazu könnte aber auch die Gantry in dieser Richtung verschoben werden. Sowohl bei der Rotation der Strahlenquelle um den Untersuchungsbereich als auch bei der Verchiebung des Untersuchungsbereichs in bezug auf die Gantry 1 kommt es nur auf die Relativbewegung an; beispielsweise könnte statt des Patientenlagerungstisches die Gantry in der entgegengesetzten Richtung verschoben werden. Wenn die Motoren 5 und 2 gleichzeitig laufen, beschreiben die Strahlenquelle S und die Detektoreinheit 16 eine Trajektorie in Form einer Helix relativ zum Untersuchungsbereich 13. Wenn hingegen der Motor 5 für den Vorschub in z-Richtung stillsteht und der Motor 2 die Gantry 1 rotieren lässt, ergibt sich eine kreisförmige Trajektorie für die Strahlenquelle S und die Detektoreinheit 16 relativ zum Untersuchungsbereich 13.

Die beiden Motoren 2 und 5, der Bildverarbeitungsrechner 10, die Strahlenquelle S und der Transfer der CT-Daten von der Detektoreinheit 16 zum Bildverarbeitungsrechner 10 werden von einer Steuereinheit 7 gesteuert.

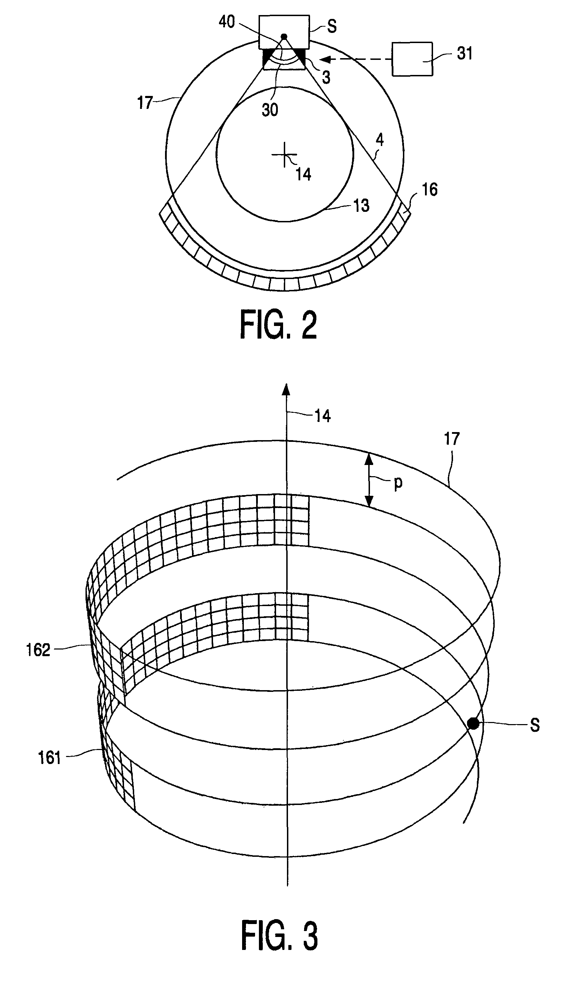

Fig. 2 stellt die Anordnung rein schematisch in einer zur Rotationsachse senkrechten Ebene dar. Die zuvor enwähnte Helix geht dann in einen Kreis 17 über und die Rotationsachse 14 in einen Punkt, den Mittelpunkt dieses Kreises. Das von der Strahlenquelle S emittierte Strahlenbündel 4 durchsetzt u. a. eine in dem Gehäuse der Kollimator-Anord nung 3 befindliche Blendenanordnung 30, bevor es in den Untersuchungsbereich 13 eintritt. Eine von der Steuereinheit 7 (Fig. 1) gesteuerte Wechseleinrichtung 31 gestattet es, die verschiedenen Komponenten im Gehäuse der Kollimator-Anordnung 3 bei Bedarf aus dem Strahlengang zu entfemen.

Fig 3 stellt die geometrischen Verhältnisse bei einer ersten Ausführungsform der Erfindung dar, wobei - ebenso wie in den weiteren Zeichnungen - die Abmessungen in Richtung der Rotationsachse im Vergleich zu den Abmessungen in den dazu senkrechten Richtungen vergrößert dargestellt sind. Man erkennt die Trajektorie, die die Strahlenquelle und der Untersuchungsbereich relativ zueinander beschreiben und die die Form einer Helix 17 hat. Benachbarte Windungen der Helix 17 haben voneinander den Abstand p. Außerdem ist die Position der Strahlenquelle S auf der Helix für einen bestimmten Zeitpunkt dargestellt sowie die beiden Flächen auf der Helix, auf die die Strahlenquelle S die beiden Detektorsegmente 161 und 162 projiziert, aus denen sich die Detektoreinheit 16 bei dieser ersten Ausführungsform zusammensetzt. Man erkennt, dass die in axialer Richtung gegeneinander versetzten Ränder der Detektorsegmente (161 und 162 bzw. ihre Projektionen auf die Helix 17) genau den Zwischenraum zwischen zwei benachbarten Helixwindungen füllen, wobei zwischen dem unteren Rand des oberen Detektorsegments 162 und dem oberen Rand des unteren Detektorelements 161 ein Abstand p (entsprechend dem Abstand zweier benachbarter Windungen der Helix) frei bleibt. In der Mitte dazwischen befindet sich die Strahlenquelle S.

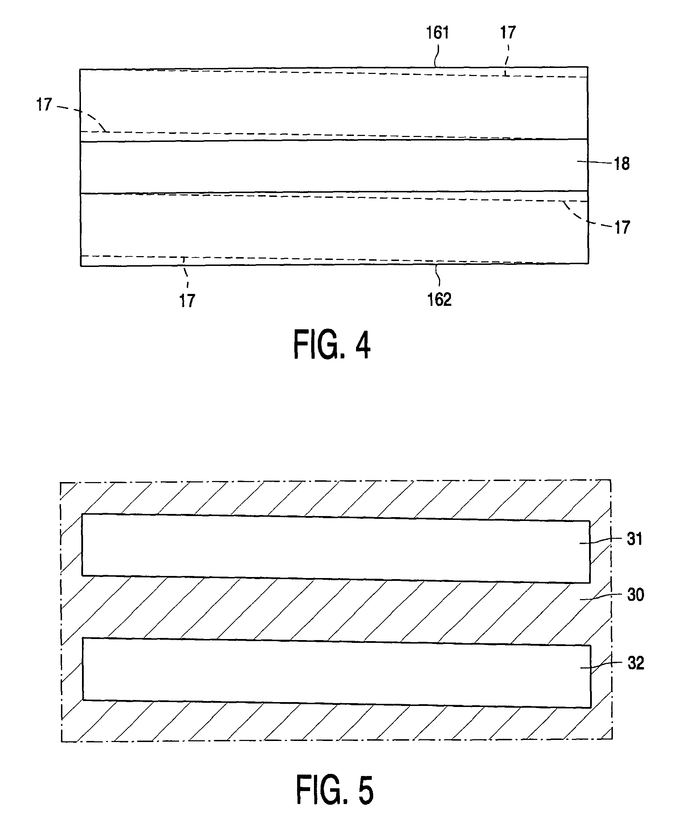

Fig. 4 stellt eine Abwicklung der aus den beiden Detektorsegmenten 161 und 162 zusammengesetzten Detektoreinheit 16 dar, in die die Projektion der Helix 17 (durch die Strahlenquelle S auf die Detektoreinheit 16) mit gestrichelten Linien eingezeichnet ist. Man erkennt, dass die Detektorsegmente 161 und 162 ein Rechteck definieren, das- im Gegensatz zu Fig. 3 - nicht mit dem durch die Projektion der Helix definierten Parallelogramm-ähnlichen Bereich identisch ist. Die Detektorsegmente (mit matrixförmig in Zeilen und Spalten angeordneten, in Fig.4 nicht näher dargestellten Detektorelementen) lassen sich in diesem Fall einfacher herstellen, und es ist lediglich dafür zu sorgen, dass die Messwerte von Detektorelementen, deren Projektion außerhalb des jeweiligen Helixsegmentes liegt, für die Rekonstruktion nicht herangezogen werden, um redundante CT-Daten zu vermeiden.

Zwischen den beiden Detektorsegmenten verbleibt ein Zwischenraum 18, dessen Breite (bzw. dessen Abmessungen in axialer Richtung) kleiner ist als die Breite eines Detektorsegments 161 oder 162. Der Raum 18 reicht aber aus, um dort die für die Detektorsegmente erforderliche Elektronik unterzubringen oder Befestigungspunkte für die Detektorsegmente bzw. für in den Zeichnungen nicht näher dargestellte Streustrahlenraster, die sich zwischen der Detektoreinheit 16 und dem Untersuchungsbereich 13 befinden und die die Streustrahlung aus dem Untersuchungsbereich weitgehend unterdrücken.

Fig. 5 zeigt eine zu Fig. 4 passende im Gehäuse der Kollimatoranordnung 3 befindliche und bei medizinischen Untersuchungen nützliche Blendenanordnung 30 aus einem für die Röntgenstrahlung praktisch undurchlässigen Material. Die Blendenanordnung enthält zwei in axialer Richtung gegeneinander versetzte Schlitze 31 und 32, durch die die Röntgenstrahlung auf die Detektorsegmente 161 und 162 fällt. Die Schlitze 31 und 32 sind dabei so geformt, dass ihre Projektion im Idealfall mit je einer Windung der Helix zusammenfällt. Dadurch wird vermieden, dass die Bereiche der Detektorsegmente 161 und 162, deren CT-Daten für die Rekonstruktion nicht benötigt werden bzw. unterdrückt werden müssen, von Röntgenstrahlung getroffen werden. Dadurch wird auch die Strahlenbelastung klein gehalten.

Durch die Blende werden so viele Strahlenkegel erzeugt, wie Detektoisegmente vorhanden sind - im vorliegenden Fall zwei. Zwischen je zwei dieser Strahlenbündel verbleibt ein von Strahlung freier Raum. Im Sinne der Erfindung wird auch ein Strahlenbündel, das sich aus mehreren solcher im Abstand zueinander verlaufender Strahlenkegel zusammensetzt als kegelförmiges Strahlenbünde bezeichnet.

Die Rekonstruktion erfolgt analog zu dem in der DE-A 198 35 296 beschriebenen Verfahren. Während bei diesem Verfahren aber sowohl bei der Filterung als auch bei der Rückprojektion der gefilterten Daten ein Winkelbereich von -270° bis +270° voll ausgenutzt wird, werden bei der vorliegenden Erfindung die CT-Daten für den Winkelbereich von-90° bis +90° Null gesetzt und die CT-Daten für die Winkelbereiche von -270° bis -90° und von +90° bis +270° mit einem um das 1,5 -fache erhöhten Gewicht zur Rekonstruktion herangezogen.

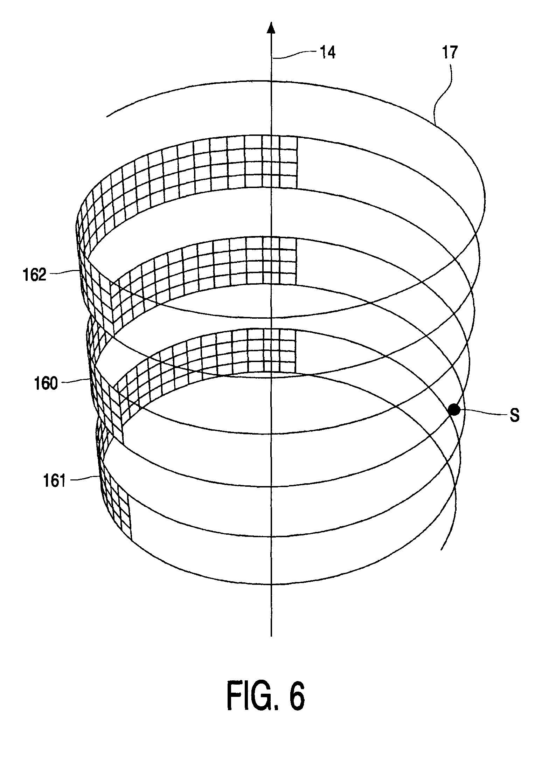

Fig 6 zeigt die geometrischen Verhältnisse bei einer bevorzugten Ausführungsform. Die Detektoreinheit besteht dabei aus drei Detektorsegmenten 161, 160 und 162, deren Projektion jeweils den Zwischenraum zweier benachbarter Windungen der Helix 17 bedecken. Zwischen dem mittleren Detektorsegment 160 einerseits und dem unteren Detektorsegment 161 bzw. dem oberen Detektorsegment 162 andererseits bleibt dabei jeweils ein Zwischenraum, der genauso breit ist wie die einzelnen Detektorsegmente. Es handelt sich somit um eine Detektoreinheit, deren Gesamtabmessungen in axialer Richtung 5p beträgt und die sich aus drei Detektorsegmenten der Breite p zusammensetzt, die im Abstand p voneinander angeordnet sind Wie in Verbindung mit Fig. 4 erläutert, kann auch hierbei die Abwicklung der einzelnen Detektorsegmente wiederum die Form eines Rechtecks haben.

Der Vorteil dieser Ausführungsform besteht darin, dass die zur Rotationsachse 14 senkrechte Ebene, in der sich die Strahlenquelle S befindet, das mittlere Detektorsegment 160 schneidet. Infolgedessen kann dieses mittlere Detektorsegment zur Akquisition von CT-Daten benutzt werden, wenn der Untersuchungsbereich statt entlang einer helixförmigen Trajektorie entlang einer kreisförmigen Trajektorie abgetastet wird.

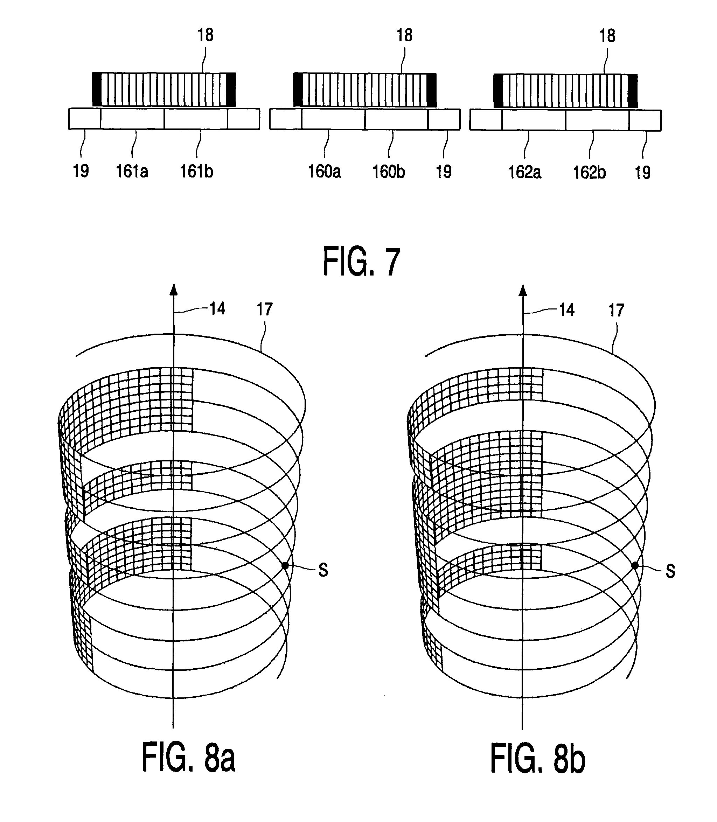

Fig. 7 zeigt einen Schnitt durch die in Fig 6 im Prinzip dargestellte Anordnung in einer die Rotationsachse enthaltenden Ebene. Die Helix ist dabei nicht dargestellt. Man erkennt, dass die Detektorsegmente 160, 161 und 162 aus je zwei Teilen 160a und 160b, 161a und 161b, 162a und 162b bestehen. Diese Teile sind so zusammengefügt, dass die strahlenempfindlichen Bereiche mit den einzelnen Detektorelementen unmittelbar aneinander angrenzen, während die Elektronik 19 zur Verarbeitung der Signale der Detektorelemente sich an den voneinander abgewandten Rändern der Teile (z. B. 160a, 160b) befindet.

Vor jedem Detektorsegment befindet sich ein Streustrahlenraster 18. Dieses ist mit senkrecht zur Zeichenebene der Fig. 7 verlaufenden Lamellen versehen, die auf die Strahlenquelle ausgerichtet sind und Streustrahlung unterdrücken, die im Untersuchung bereich erzeugt wird und unter einem anderen Einfallswinkel auf das Streustrahlenraster 18 trifft als die von der Strahlenquelle emittierte Strahlung Der Zwischenraum zwischen zwei Detektorsegmenten kann außer für die Elektronik zusätzlich noch für eine - nicht näher dargestellte - stabile Befestigung der Streustrahlenraster und der Detektorsegmente an der Gantry benutzt werden.

Die Tatsache, dass man mit den CT-Daten des mittleren Detektorsegments einerseits und den CT-Daten der beiden äußeren Detektorsegmente andererseits ein vollständiges CT-Bild rekonstruieren kann, lässt sich für computertomographische "dual energy" Untersuchungen ausnutzen. Dabei werden die beiden äußeren Detektorsegmente 161 und 162 mit Strahlung einer anderen Energie bestrahlt als das mittlere Detektorsegment. Zu diesem Zweck wird in der Kolimatoranordnung ein Filter 40 (vgl. Fig 2) verwendet, das die Strahlung unterschiedlich aufhärtet. Beispielsweise kann das Filter so gestaltet sein, dass die Strahlung zum mittleren Detektorsegment völlig unbeeinflusst bleibt (d. h. in diesem Bereich enthält das Filter eine Öffnung, die die Strahlung unbeeinflusst passieren lässt), während die Strahlung zu den beiden äußeren Detektorsegmenten durch ein geeignetes Filtermaterial mit einer geeigneten Dicke in einer bestimmten Weise aufgehärtet wird. Der Vorteil einer solchen "dual energy" Untersuchung in Verbindung mit der Erfindung besteht darin, dass die dafür erforderlichen CT-Daten gleichzeitig (und nicht nacheinander) akquiriert werden können.

Die Fig 8 a und 8 b stellen zwei verschiedene Ausführungsformen dar, bei denen die Projektion der äußeren Ränder der Detektoreinheit eine Strecke von 7p bedeckt. Die Detektoreinheit besteht wiederum aus drei Detektorsegmenten - einem zentralen Detektorsegment 160 und zwei gleich breiten äußeren Segmenten 161 und 162.

Während aber bei der Ausführungsform nach Fig 8a das mittlere Detektorsegment (in der Projektion) nur die Breite p hat und die beiden äußeren Detektorsegmente je die Breite 2p haben, hat bei der Ausführungsform nach Fig. 8b das mittlere Detektorsegment die Breite 3p und die beiden äußeren Detektorsegmente 161 und 162 die Breite p. Diese Ausführungsform eignet sich besser für Untersuchungen mit kreisförmiger Trajektorie.

Die Projektion der äußeren Ränder der Detektoreinheit kann sich auch über mehr als 7p erstrecken (allgemein über (2n+1)p, wobei n eine ganze Zahl ist), und die Detektoreinheit kann in bis zu n+1 Detektorsegmente aufgeteilt sein. Die Detektorsegmente sollen dabei symmetrisch zu der Strahlenquelle S (d.h. deren Fokus, von dem die Strahlung ausgeht) bzw. zu dem Punkt angeordnet sein, in dem das Lot von S auf die Rotationsachse 14 den durch die Helix definierten Zylinder durchstößt.

## Patentansprüche

1. Computertomograph mit
- einer Abtasteinheit, die eine Strahlenquelle (S) und eine damit verbundene Detektoreinheit (16) zur Erfassung eines von der Strahlenquelle (S) emittierten, kegelförmigen Strahlenbündels nach dem Durchgang durch einen Untersuchungsbereich (13) bzw. durch ein darin befindliches Objekt umfasst,
- einer Antriebsanordnung (2, 5) zur Erzeugung einer eine Rotation um eine Rotationsachse (14) und einen Vorschub parallel zur Rotationsachse umfassenden Relativbewegung in Form einer Helix zwischen der Abtasteinheit (S, 16) und dem Untersuchungsbereich (13) bzw. dem Objekt,
- wobei die Projektion der in axialer Richtung gegeneinander versetzten äußeren Ränder der Detektoreinheit auf die Helix eine Strecke von (2n+1)p einschließt, wobei n eine ganze Zahl ≥ 1 ist und p dem axialen Versatz zweier benachbarter Windungen der Helix entspricht,
**dadurch gekennzeichnet, dass**
die Detektoreinheit mehrere räumlich voneinander getrennte und in axialer Richtung gegeneinander versetzte eine Vielzahl von Detektorelementen umfassende Detektorsegmente (161, 162) im Abstand voneinander umfasst, wobei zwischen den Detektorsegmenten (161, 162) ein Zwischenraum (18) verbleibt, und jedes Detektorsegment (161, 162) so angeordnet und gestaltet ist, dass seine Projektion auf die Helix mindestens zwei benachbarte Windungen der Helix bedeckt.

2. Computertomograph nach Anspruch 1,
**dadurch gekennzeichnet,**
**dass** n=1 beträgt und dass die Detektoreinheit zwei Detektorsegmente beiderseits einer zur Rotationsachse senkrechten und die Strahlenquelle enthaltenden Ebene umfasst.

3. Computertomograph nach Anspruch 1,
**dadurch gekennzeichnet,**
**dass** n mindestens 2 beträgt und dass die Detektoreinheit mindestens drei Detektorsegmente umfasst, von denen sich eines in einer zur Rotationsachse senkrechten und die Strahlenquelle enthaltenden Ebene und die restlichen je zur Hälfte auf je einer Seite dieser Ebene befinden.

4. Computertomograph nach Anspruch 1,
**dadurch gekennzeichnet,**
**dass** zwischen der Strahlenquelle und dem Untersuchungsbereich eine Blendenanordnung angeordnet ist, die die Zwischenräume zwischen den Detektorsegmenten von der Strahlung der Strahlenquelle abschirmt.

5. Computertomograph nach Anspruch 3,
**dadurch gekennzeichnet,**
**dass** zwischen der Strahlenquelle und dem Untersuchungsbereich eine Filteranordnung angeordnet ist, deren Absorptionseigenschaften zur Erzeugung von Strahlung mit zwei unterschiedlicher Energiespektren in axial versetzten Bereichen eine solche Ortsabhängigkeit aufweisen, dass eine erste Gruppe von Detektorsegmenten Strahlung mit einem ersten Energiespektrum und eine zweite Gruppe von Detektorsegmenten Strahlung mit einem zweiten Energiespektrum empfängt.

## Claims

1. A computer tomograph, which includes
- a scanning unit which includes a radiation source (S) and a detector unit (16) which is connected thereto in order to detect a cone-shaped radiation beam, emitted by the radiation source (S), after its passage through an examination zone (13) or an object present therein,
- a drive device (2, 5) for generating a relative movement in the form of a helix, comprising a rotation about an axis of rotation (14) and a displacement parallel to the axis of rotation, between the scanning unit (S, 16) and the examination zone (13) or the object,
- the projection of the outer edges of the detector unit, being mutually offset in axial direction, onto the helix including a path of (2n+1)p, where n is an integer ≥ 1 and p corresponds to the axial offset of two neighboring turns of the helix,
**characterized in that** the detector unit includes a plurality of mutually spatially separated detector segments (161, 162), which are mutually offset in axial direction and comprise a plurality of detector elements, a clearance (18) remaining between the detector segments (161, 162) and each detector segment (161, 162) being arranged and shaped in such a manner that its projection onto the helix covers at least two neighboring turns of the helix.

2. A computer tomograph as claimed in claim 1, **characterized in that** n = 1 and that the detector unit includes two detector segments that are situated to both sides of a plane that extends perpendicularly to the axis of rotation and contains the radiation source.

3. A computer tomograph as claimed in claim 1, **characterized in that** n amounts to at least 2 and that the detector unit includes at least three detector segments, one of which is situated in a plane that extends perpendicularly to the axis of rotation and contains the radiation source whereas a respective half of the remaining segments is situated each one one side of this plane.

4. A computer tomograph as claimed in claim 1, **characterized in that** a diaphragm device is arranged between the radiation source and the examination zone in such a manner that it shields the clearances between the detector segments from the radiation from the radiation source.

5. A computer tomograph as claimed in claim 3, **characterized in that** between the radiation source and the examination zone there is arranged a filter device whose absorption properties exhibit such a spatial dependence so as to generate radiation of two different energy spectra in axially offset areas such that a first group of detector segments receives radiation having a first energy spectrum, whereas a second group of detector segments receives radiation having a second energy spectrum.

## Revendications

1. Tomographe informatisé avec
- une unité de lecture qui comprend une source de rayons (S) et une unité de détecteur (16) reliée à celle-ci pour la saisie d'une faisceau de rayons de forme conique émis par la source de rayons (S) après le passage par une zone d'examen (13) ou par un objet qui s'y trouve,
- un dispositif d'entraînement (2, 5) pour la production d'un mouvement relatif comprenant une rotation autour d'un axe de rotation (14) et une avance parallèlement à l'axe de rotation sous la forme d'une hélice entre l'unité de balayage (S, 16) et la zone d'examen (13) ou l'objet,
- la projection des bords extérieurs de l'unité de détecteur décalés l'un par rapport à l'autre en direction axiale renfermant sur l'hélice un tronçon de (2n+1)p, n étant un nombre entier ≥ 1 et p le déport axial de deux enroulements voisins de l'hélice,
**caractérisé en ce que**
l'unité de détecteur comprend plusieurs segments détecteurs (161, 162) comprenant plusieurs éléments détecteurs, détectés les uns par rapport aux autres en direction axiale et séparés spatialement les uns des autres, un intervalle (18) subsistant entre les segments détecteurs (161, 162) et chaque segment détecteur (161, 162) étant disposé et conçu de telle sorte que sa projection couvre au moins deux enroulements voisins de l'hélice sur l'hélice.

2. Tomographe informatisé selon la revendication 1,
**caractérisé en ce**
**que** n = 1 et que l'unité détecteur comprend deux segments détecteurs de part et d'autre d'un plan perpendiculaire à l'axe de rotation et contenant la source de rayonnement.

3. Tomographe informatisé selon la revendication 1,
**caractérisé en ce**
**que** n s'élève à au moins 2 et que l'unité de détecteur comprend au moins trois segments détecteurs dont l'un se trouve dans un plan perpendiculaire à l'axe de rotation et contenant la source de rayons et les autres se trouvent à raison d'une moitié chacun sur un côté respectif de ce plan.

4. Tomographe informatisé selon la revendication 1,
**caractérisé en ce**
**qu'**un dispositif à diaphragme qui protège les intervalles entre les segments détecteurs du rayonnement de la source de rayons est disposé entre la source de rayons et la zone d'examen.

5. Tomographe informatisé selon la revendication 3,
**caractérisé en ce**
**qu'**il est disposé entre la source de rayons et la zone d'examen un dispositif de filtrage dont les propriétés d'absorption présentent pour la production du rayonnement avec deux spectres énergétiques différents dans des zones décalées axialement une dépendance spatiale telle qu'un premier groupe de segments détecteurs reçoit un rayonnement avec un premier spectre énergétique et un deuxième groupe de segments détecteurs un rayonnement avec un deuxième spectre énergétique.
